# EUROPEAN PATENT APPLICATION

(11) **EP 2 103 269 A1**
(43) Date of publication of application: **23.09.2009**
(21) Application number: 09003813.4
(22) Date of filing: 17.03.2009
(51) Int. Cl.: A61B 18/16

(54) **Variable capacitive electrode pad**

(30) Priority: 17.03.2008 US 37243 P; 03.03.2009 US 396814
(71) Applicant: Tyco Healthcare Group, LP, North Haven CT 06473 (US)
(72) Inventor: Behnke, Robert J., Erie, CO 80516 (US); Keppel, David, Longmont, CO 80501 (US)
(74) Representative: HOFFMANN EITLE

(57) **Abstract**

An electrosurgical system is disclosed. The system includes one or more variable capacitive pads including one or more pairs of split electrodes arranged in a capacitive configuration, wherein the pair of split electrodes is adapted to connect to an electrosurgical generator. The system also includes a return electrode monitoring system coupled to the pair(s) of split electrodes and is configured to map an initial capacitance between the split electrodes with substantially full adherence of the variable capacitive pad to the patient and determine an adherence factor of the variable capacitance pad as a function of a change in capacitance between the pair(s) of split electrodes with respect to the map of the initial capacitance indicative of substantially full adherence.

## Description

### BACKGROUND

### Technical Field

The present disclosure relates to electrosurgical apparatuses, systems and methods. More particularly, the present disclosure is directed to electrosurgical systems utilizing one or more capacitive return electrode pads configured to monitor contact quality thereof.

### Background of Related Art

Energy-based tissue treatment is well known in the art. Various types of energy (e.g., electrical, ultrasonic, microwave, cryogenic, heat, laser, etc.) are applied to tissue to achieve a desired result. Electrosurgery involves application of high radio frequency electrical current to a surgical site to cut, ablate, coagulate or seal tissue. In monopolar electrosurgery, the active electrode is typically part of the surgical instrument held by the surgeon and applied to the tissue to be treated. A patient return electrode is placed remotely from the active electrode to carry the current back to the generator and safely disperse current applied by the active electrode.

The return electrodes usually have a large patient contact surface area to minimize heating at that site. Heating is caused by high current densities which directly depend on the surface area. A larger surface contact area results in lower localized heat intensity. Return electrodes are typically sized based on assumptions of the maximum current utilized during a particular surgical procedure and the duty cycle (i.e., the percentage of time the generator is on).

The first types of return electrodes were in the form of large metal plates covered with conductive jelly. Later, adhesive electrodes were developed with a single metal foil covered with conductive jelly or conductive adhesive. However, one issue with these adhesive electrodes was that if a portion peeled from the patient, the contact area of the electrode with the patient decreased, thereby increasing the current density at the adhered portion and, in turn, increasing the heating at the tissue. This risked burning the patient in the area under the adhered portion of the return electrode if the tissue was heated beyond the point where circulation of blood could cool the skin.

To address this problem various return electrodes and hardware circuits, generically called Return Electrode Monitors (REMs) and Return Electrode Contact Quality Monitors (RECQMs), were developed. Such systems relied on measuring impedance at the return electrode to calculate a variety of tissue and/or electrode properties. These systems were only configured to measure changes in impedance of the return electrodes to detect peeling. Further, the systems were only designed to work with conventional resistive return electrode pads. Still, other systems are configured to detect a fault (e.g., defect) in the return electrode pad (e.g., in the dielectric material) based on a detected phase difference between the current and the voltage of the electrosurgical energy. One such system is disclosed in U.S. Patent Application 61/037,210 entitled "SYSTEM AND METHOD FOR DETECTING A FAULT IN A CAPACITIVE RETURN ELECTRODE FOR USE IN ELECTROSURGERY," which is being filed with the United States Patent and Trademark Office concurrently herewith.

### SUMMARY

The present disclosure relates to an electrosurgical variable capacitive return electrode pad. The variable capacitive pad includes one or more pairs of split return electrodes arranged in a capacitive configuration. This allows a generator having a return electrode monitoring system to measure capacitance between the split return electrodes and map the initial capacitance with full adherence of the variable capacitive pad to the patient. The return electrode monitoring system then monitors the capacitance between the split return electrodes and correlates the change therein with the contact quality of the variable capacitive pad based on the initial mapping.

According to one aspect of the present disclosure an electrosurgical system is disclosed. The system includes one or more variable capacitive pads including a pair of split electrodes arranged in a capacitive configuration, wherein the pair of split electrodes is configured to return electrosurgical energy to a generator. The system also includes a return electrode monitoring system coupled to the at least one pair of split electrodes and is configured to map an initial capacitance between the at least one pair of split electrodes with substantially full adherence of the at least one variable capacitive pad to the patient and determines an adherence factor of the variable capacitance pad as a function of a change in capacitance between the at least one pair of split electrodes with respect to the map of the initial capacitance indicative of substantially full adherence.

According to another aspect of the present disclosure a return electrode monitoring system is disclosed. The system includes a variable capacitive pad having a pair of split electrodes arranged in a capacitive configuration, wherein the pair of split electrodes is configured to return electrosurgical energy to a generator. The system also includes a detection circuit coupled to the pair of split electrodes and configured to measure capacitance therebetween. The detection circuit also maps initial capacitance between the at least one pair of split electrodes with substantially full adherence of the variable capacitive pad to the patient and determines contact quality of the variable capacitive pad based on the change in capacitance between the pair of split electrodes.

A method for monitoring a variable capacitive pad is also contemplated by the present disclosure. The method includes the step of providing a monitor signal waveform to a variable capacitive pad having a pair of split electrodes arranged in a capacitive configuration, wherein the pair of split electrodes is configured to return electrosurgical energy to a generator. The method also includes the steps of measuring a property of the monitor signal waveform and determining capacitance between the pair of split electrodes based on the property of the monitor signal waveform.

### BRIEF DESCRIPTION OF THE DRAWINGS

Various embodiments of the present disclosure are described herein with reference to the drawings wherein:
Fig. 1 is a schematic block diagram of an electrosurgical system according to the present disclosure;
Fig. 2 is a schematic block diagram of a generator according to one embodiment of the present disclosure;
Figs. 3A - 3D are top cross-sectional views of multi-sectioned capacitive return electrode pads in accordance with the present disclosure; and
Fig. 4 is a flow chart diagram of a method according to one embodiment of the present disclosure.

### DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail.

A capacitive return electrode pad can safely return more current than a return electrode pad incorporating a resistive design. However, conventional capacitive return electrode pads are not configured to couple with a return electrode monitoring ("REM") system. The REM system monitors the adherence of the return electrode pad to the patient by measuring the impedance and/or current between one or more split pads. Split pad designs have been incorporated into resistive return electrode pads but previously were not included in capacitive return electrode designs due to the increased impedance of these electrode pads.

The present disclosure provides for a variable capacitive return electrode pad incorporating capacitive and return electrode monitoring technologies. More specifically, the capacitive return pad according to embodiments of the present disclosure includes a plurality of split electrodes that create a capacitance therebetween. When the capacitive return pad is in contact with the patient, capacitance between the split electrodes increases. This capacitance, and any changes therein, is used to monitor the contact area between the patient and the capacitive return pad.

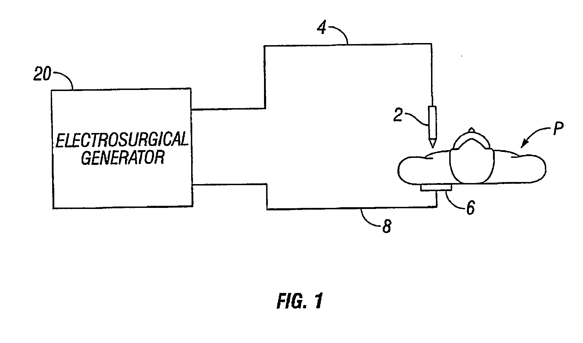

Fig. 1 is a schematic illustration of an electrosurgical system according to one embodiment of the present disclosure. The system includes an electrosurgical instrument 2 having one or more electrodes for treating tissue of a patient P. The instrument 2 is a monopolar instrument including one or more active electrodes (e.g., electrosurgical cutting probe, ablation electrode(s), etc.). Electrosurgical RF energy is supplied to the instrument 2 by a generator 20 via an electrosurgical cable 4, which is connected to an active output terminal, allowing the instrument 2 to coagulate, ablate and/or otherwise treat tissue. The energy is returned to the generator 20 through a return electrode pad 6 via a return cable 8. The system may include a plurality of return electrodes pads 6 that are arranged to minimize the chances of tissue damage by maximizing the overall contact area with the patient P. In addition, the generator 20 and the return electrode 6 may be configured for monitoring so-called "tissue-to-patient" contact to insure that sufficient contact exists therebetween to further minimize chances of tissue damage.

The generator 20 includes input controls (e.g., buttons, activators, switches, touch screen, etc.) for controlling the generator 20. In addition, the generator 20 may include one or more display screens for providing the user with variety of output information (e.g., intensity settings, treatment complete indicators, etc.). The controls allow the user to adjust power of the RF energy, waveform, and other parameters to achieve the desired waveform suitable for a particular task (e.g., coagulating, tissue sealing, intensity setting, etc.). The instrument 2 may also include a plurality of input controls that may be redundant with certain input controls of the generator 20. Placing the input controls at the instrument 2 allows for easier and faster modification of RF energy parameters during the surgical procedure without requiring interaction with the generator 20.

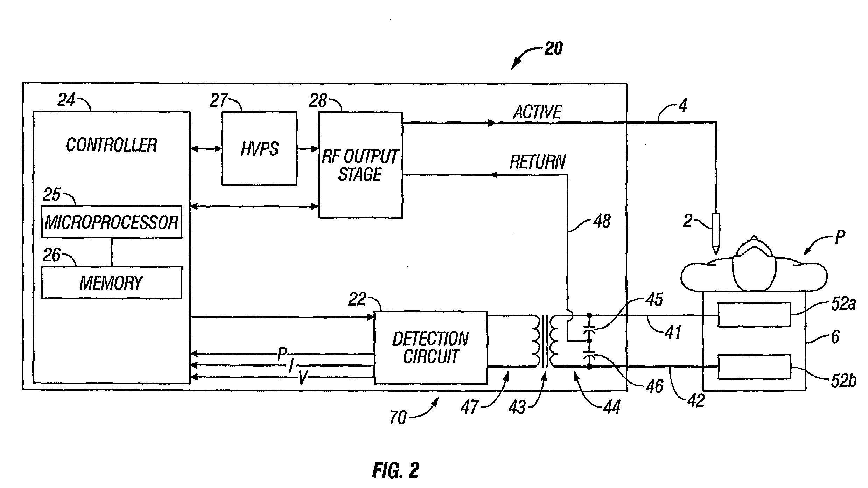

Fig. 2 shows a schematic block diagram of the generator 20 having a controller 24, a high voltage DC power supply 27 ("HVPS") and an RF output stage 28. The HVPS 27 provides high voltage DC power to an RF output stage 28, which then converts high voltage DC power into RF energy and delivers the RF energy to the active electrode. In particular, the RF output stage 28 generates sinusoidal waveforms of high RF energy. The RF output stage 28 is configured to generate a plurality of waveforms having various duty cycles, peak voltages, crest factors, and other suitable parameters. Certain types of waveforms are suitable for specific electrosurgical modes. For instance, the RF output stage 28 generates a 100% duty cycle sinusoidal waveform in cut mode, which is best suited for ablating, fusing and dissecting tissue, and a 1-25% duty cycle waveform in coagulation mode, which is best used for cauterizing tissue to stop bleeding.

The controller 24 includes a microprocessor 25 operably connected to a memory 26, which may be volatile type memory (e.g., RAM) and/or non-volatile type memory (e.g., flash media, disk media, etc.). The microprocessor 25 includes an output port that is operably connected to the HVPS 27 and/or RF output stage 28 that allows the microprocessor 25 to control the output of the generator 20 according to either open and/or closed control loop schemes. The microprocessor 25 may be substituted by any suitable logic processor (e.g., control circuit) adapted to perform the calculations discussed herein.

The generator 20 may include a sensor circuit (not explicitly shown) having suitable sensors for measuring a variety of tissue and energy properties (e.g., tissue impedance, tissue temperature, output current and/or voltage, etc.) and to provide feedback to the controller 24 based on the measured properties. Such sensors are within the purview of those skilled in the art. The controller 24 then signals the HVPS 27 and/or RF output stage 28, which then adjust DC and/or RF power supply, respectively. The controller 24 also receives input signals from the input controls of the generator 20 or the instrument 2. The controller 24 utilizes the input signals to adjust power outputted by the generator 20 and/or performs other control functions thereon.

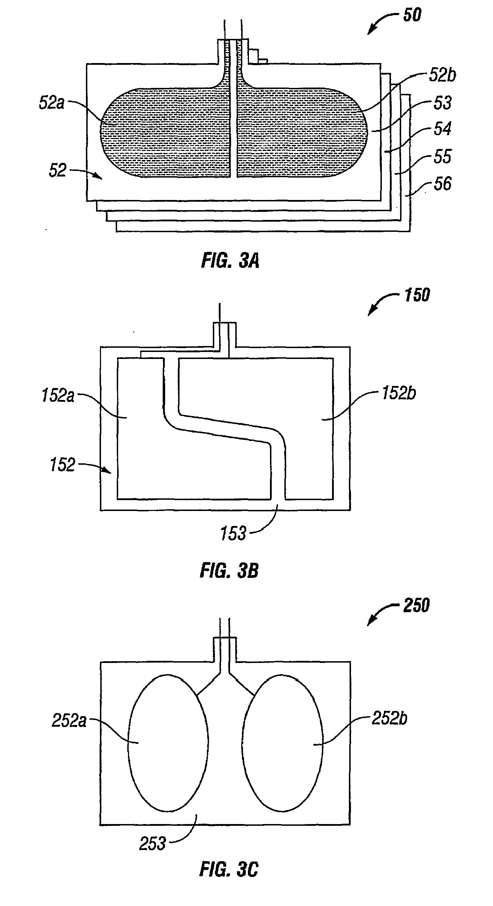

Referring now to Fig. 3A, the return electrode pad 6 is embodied as a variable capacitive pad ("VCP") 50 for providing a return path for electrosurgical current and monitoring surface impedance and capacitance according to the present disclosure. While the VCP 50 is depicted as having a general rectangular shape, it is within the scope of the disclosure for the VCP 50 to have any suitable regular or irregular shape.

VCP 50 includes a carrier layer 53 having one or more layers, such as, a backing layer 52, a heat distribution layer 54, a passive cooling layer 55, and an attachment layer 56. An active cooling layer (not explicitly shown) or any other suitable insulating layer or combination above layers 53, 54, 55, and 56 may also be included.

The attachment layer 56 is disposed on a patient-contacting surface of the VCP 50 and may be formed from an adhesive material (not explicitly shown) which may be, but is not limited to, a polyhesive adhesive, a Z-axis adhesive, a water-insoluble, hydrophilic, pressure-sensitive adhesive, or any combinations thereof, such as POLYHESIVE^{™} adhesive manufactured by Valleylab, a division of Tyco Healthcare of Boulder, Colorado. The adhesive may be conductive or dielectric. The attachment layer 56 ensures an optimal surface contact area between the electrosurgical return electrode pad 6 and the patient "P," which minimizes the risk of damage to tissue. In another embodiment, the VCP 50 may be reusable and have a sufficiently large surface area so that the VCP 50 may be used without the attachment layer 56, allowing the VCP 50 to be cleaned and sanitized between uses.

The backing layer 52 supports a pair of split return electrodes 52a and 52b for positioning under a patient during electrosurgery. The backing layer 52 may be made of cloth, cardboard, non-woven or any suitable material. In one embodiment, the backing layer 52 may be formed from a dielectric material such as flexible polymer materials to enhance capacitive properties of the VCP 50. The polymer materials may be polyimide film sold under a trademark KAPTON™ and polyester film, such as biaxially-oriented polyethylene terephthalate (boPET) polyester film sold under trademarks MYLAR™ and MELINEX™. In another embodiment the backing layer 52 may act as an insulating layer between the pair of split return electrodes 52a and 52b and the attachment layer 56.

The split return electrodes 52a and 52b may be made from materials that include aluminum, copper, mylar, metalized mylar, silver, gold, stainless steel or other suitable conductive material and may be of various shapes and may be arranged in various configurations and orientations. The split configuration of the split return electrodes 52a and 52b create a measurable capacitance therebetween which may be measured by generator 20 to determine adherence of the VCP 50 to the patient "P."

More specifically, capacitive coupling between the split return electrodes 52a and 52b increases upon the initial placement of the VCP 50 in contact with the patient "P." This capacitance corresponds to full adherence of the VCP 50 to the patient. During the procedure, the VCP 50 may peel from the patient "P," thereby decreasing the adherence factor thereof. The decrease in adherence directly affects the capacitance between the split return electrodes 52a and 52b. Measuring the change in capacitance between the split return electrodes 52a and 52b, therefore, provides an accurate measurement of adherence of the VCP 50 to the patient "P." The amount of capacitance coupling or the change in capacitance coupling then may be used to insure positive patient contact or to determine adequate patient coverage of the VCP 50.

With returned reference to Fig. 2, the generator 20 includes a return electrode monitoring ("REM") system 70 having a detection circuit 22 which is coupled to the split return electrodes 52a and 52b. The VCP 50 is in contact with the patient "P" and returns the electrosurgical energy to the generator 20 via the split return electrodes 52a and 52b that are coupled to leads 41 and 42 respectively. In one embodiment, the VCP 50 may include a plurality of pairs of split electrode pads which are coupled to a corresponding number of leads. The leads 41 and 42 are enclosed in a return cable 8 and are terminated at a secondary winding 44 of a transformer 43. The leads 41 and 42 are interconnected by capacitors 45 and 46. A return lead 48 is coupled between the capacitors 45 and 46 and is adapted to return the electrosurgical energy to the RF output stage 28. The transformer 43 of the REM system 70 also includes a primary winding 47 which is connected to the detection circuit 22.

Components of the REM system 70, e.g., the transformer 43, the split return electrodes 52a and 52b, the capacitors 45 and 46, along with the detection circuit 22 form a resonant system which is adapted to resonate at a specific interrogation frequency from the controller 24. Namely, the controller 24 provides an interrogation signal at a specific interrogation frequency to the detection circuit 22. The detection circuit 22 then rectifies the interrogation signal to generate a monitor signal. The monitor signal is a constant, physiologically benign waveform (e.g., 140 kHz, 2 mA) which the detection circuit 22 applies to the split return electrodes 52a and 52b. The monitor signal thereafter passes through the patient and is returned to the circuit 22 via the split return electrodes 52a and 52b.

The returning monitor signal is modified by the capacitance of the split return electrodes 52a and 52b. More specifically, as the capacitance between the split return electrodes 52a and 52b changes due to peeling of the VCP 50 from the patient, the resonance of the detection circuit 22 with respect to other components changes as well. The change in the resonance, in turn, affects the change in amplitude of the monitor signal. Thus, the detection circuit 22 determines the magnitude of the capacitance between the split return electrodes 52a and 52b by monitoring changes in amplitude of the monitor signal waveform. The detection circuit 22 then supplies the capacitance measurement to the controller 24 which determines whether the capacitance is within a predetermined range. Initially, the controller 24 may determine an initial capacitance value corresponding to full adherence of the VCP 50. The initial capacitance value may be used as a baseline measurement of capacitive coupling between the split return electrodes 52a and 52b to determine the contact area between the patient "P" and the VCP 50. Additional measurements may be made after the VCP 50 is placed in contact with the patient "P" and prior to initiating the delivery of electrosurgical energy to patient tissue. Subsequent measurements may be made after commencement of electrosurgical energy delivery to determine any degradation in contact quality or change in a characteristic of patient contact. If the capacitance is out of range, thereby indicating excessive peeling of the return electrode pad 6, the generator 20 issues an alarm (e.g., audibly, visually, etc. via the controller 24) and/or the controller 24 adjusts the output of the generator 20 (e.g., terminates RF supply).

Figs. 3B-3D illustrate additional embodiments of the VCP 150, 250, and 300, respectively, and the corresponding arrangement of the split return electrodes. More specifically, Figs. 3B and 3C illustrate that the split return electrodes 152a, 152b and 252a, 252b may be of various shapes and sizes designed to maximize capacitive coupling between the VCP 150, 250 and the patient "P." In Fig. 3B, the VCP 150 includes L-shaped split return electrodes 152a and 152b arranged in a reverse interlocking configuration to maximize contact area with the patient "P." In Fig. 3C, the VCP 250 includes oval shaped split return electrodes 252a and 252b.

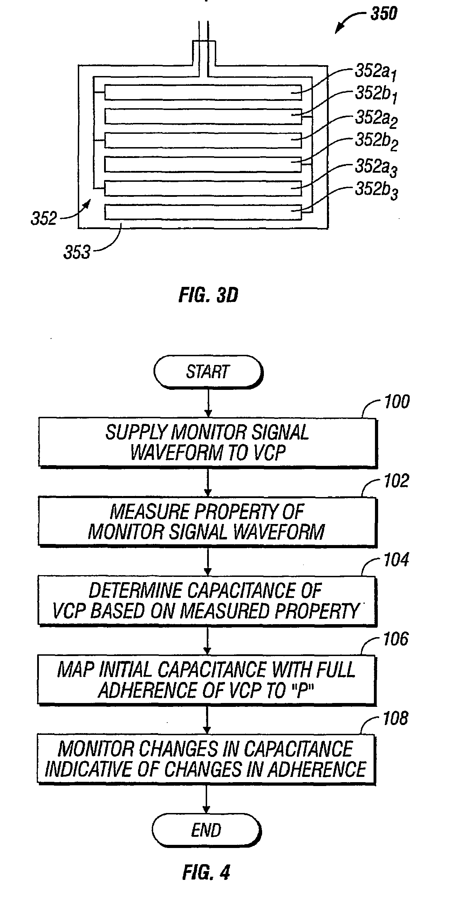

In another embodiment illustrated in Fig. 3D, the VCP 350 may include a plurality of split return electrodes arranged in an interweaving pattern. The VCP 350 includes three (3) split return electrodes for each pair, namely, 352a₁,352a₂, and 352a₃ and 352b₁, 352a₂, and 352b₃. The split return electrodes 350a₁ - 350a₃ are coupled to the return lead 41 and the split return electrodes 350b₁ - 350b₃ are coupled to the return lead 42 with each pair of the split electrodes being arranged in a sequential manner. Having multiple pairs of return electrode pads within the VCP 350 allows for fault protection in case one or more pairs of the return electrode pads fail.

Fig. 4 illustrates a method for monitoring adherence of the VCP 50 to the patient "P." In step 100, the controller 24 supplies a interrogation signal to the REM system 70. The controller 24 rectifies the interrogation signal and supplies a monitor signal waveform across the split return electrodes 52a and 52b. In step 102, the detection circuit 22 measures the current and voltage of the monitor signal waveform, which are used by the controller 24 to determine the phase of the monitor signal waveform with respect to frequency. In step 104, the controller 24 determines the reactance as a function of the voltage, current and phase values with respect to frequency. The phase of the monitor signal waveform may be determined by sweeping the interrogation signal across the resonance range of the REM system 70. This allows for correlation of phase responses with respect to multiple frequency interrogation signals.

The reactance is used to determine the capacitance of the split return electrodes 52a and 52b. In step 106, prior to the start of the electrosurgical procedure, the capacitance is mapped with respect to full adherence of the return electrode pad 6. In step 108, as the procedure commences, the capacitance is monitored and is used to determine the adherence of the VPC 50.

Other methods for monitoring contact quality of the return pad to the patient include utilizing a sensor to communicate parameters such as capacitance, to the electrosurgical generator. One such method is disclosed in United States Patent Application Serial No. 11/800,687 entitled "CAPACITIVE ELECTROSURGICAL RETURN PAD WITH CONTACT QUALITY MONITORING," filed May 7, 2007.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. An electrosurgical system comprising:
at least one variable capacitive pad including at least one pair of split electrodes arranged in a capacitive configuration, wherein the at least on pair of split electrodes is adapted to connect to an electrosurgical generator; and
a return electrode monitoring system coupled to the at least one pair of split electrodes and configured to map an initial capacitance between the at least one pair of split electrodes with substantially full adherence of the at least one variable capacitive pad to the patient and determine an adherence factor of the variable capacitance pad as a function of a change in capacitance between the at least one pair of split electrodes with respect to the map of the initial capacitance indicative of substantially full adherence.

2. An electrosurgical system according to claim 1, wherein the at least one pair of split electrodes are L-shaped and are arranged in a reverse interlocking configuration.

3. An electrosurgical system according to claim 1 or 2, wherein the at least one variable capacitive pad includes a plurality of split electrodes, each of the split electrodes having a first electrode coupled to a first return lead and a second electrode coupled to a second return lead.

4. An electrosurgical system according to claim 1, 2 or 3, wherein the conductive material is selected from the group consisting of silver, copper, gold and stainless steel.

5. A return electrode monitoring system comprising:
at least one variable capacitive pad including at least one pair of split electrodes arranged in a capacitive configuration, wherein the at least one pair of split electrodes is adapted to connect to an electrosurgical generator; and
a detection circuit coupled to the at least one pair of split electrodes and configured to measure capacitance therebetween, wherein the detection circuit maps initial capacitance between the at least one pair of split electrodes with substantially full adherence of the at least one variable capacitive pad to the patient and determines contact quality of the at least one variable capacitive pad based on a change in capacitance between the at least one pair of split electrodes.

6. A return electrode monitoring system according to claim 5, wherein the at least one pair of split electrodes are L-shaped and are arranged in a reverse interlocking configuration.

7. A return electrode monitoring system according to claim 5 or 6, wherein the at least one variable capacitive pad includes a plurality of split electrodes, each of the split electrodes having a first electrode coupled to a first return lead and a second electrode coupled to a second return lead.

8. A return electrode monitoring system according to claim 5, 6 or 7, wherein the conductive material is selected from the group consisting of silver, copper, gold and stainless steel.

9. A method for monitoring at least one variable capacitive pad, comprising the steps of:
providing a monitor signal waveform to at least one variable capacitive pad having at least one pair of split electrodes arranged in a capacitive configuration, wherein the at least one pair of split electrodes is adapted to connect to an electrosurgical generator;
measuring at least one property of the monitor signal waveform; and
determining a capacitance between the at least one pair of split electrodes based on the at least one property of the monitor signal waveform.

10. A method according to claim 9, further comprising the step of:
mapping an initial capacitance between the at least one pair of split electrodes with substantially full adherence of the at least one variable capacitive pad to the patient.

11. A method according to claim 10, further comprising the step of:
calculating an adherence factor of the variable capacitive pad as a function of a change in capacitance between the at least one pair of split electrodes with respect to the mapping of initial capacitance indicative of substantially full adherence.

12. A method according to claim 9, 10 or 11, wherein the at least one pair of split electrodes are L-shaped and are arranged in a reverse interlocking configuration.

13. A method according to any one of claims 9 to 12, wherein the at least one variable capacitive pad includes a plurality of split electrodes, each of the split electrodes having a first electrode coupled to a first return lead and a second electrode coupled to a second return lead.

14. A method according to any one of claims 9 to 13, wherein the conductive material is selected from the group consisting of silver, copper, gold and stainless steel.

15. A method according to any one of claims 9 to 14, further comprising the step of:
detecting at least one of a current, a voltage, and a phase with respect to the frequency of the monitor signal waveform.
